# EUROPÄISCHE PATENTSCHRIFT

(11) **EP 0 518 889 B1**
(45) Veröffentlichungstag und Bekanntmachung des Hinweises auf die Patenterteilung: **03.08.1994**
(21) Anmeldenummer: 91904378.6
(22) Anmeldetag: 27.02.1991
(51) Int. Cl.: C07C 317/28, C07C 315/02

(54) **VERFAHREN ZUR HERSTELLUNG VON 3'-AMINOPROPYL-2-SULFATOETHYLSULFON**
METHOD FOR PREPARING 3'-AMINOPROPYL-2-SULPHATOETHYLSULPHONE
PROCEDE DE PRODUCTION DE 3'-AMINOPROPYL-2-SULFATOETHYLSULFONE

(30) Priorität: 07.03.1990 DE 4007049
(43) Veröffentlichungstag der Anmeldung: 23.12.1992
(73) Patentinhaber: HOECHST AKTIENGESELLSCHAFT, 65926 Frankfurt am Main (DE)
(72) Erfinder: MEIER, Michael, D-6000 Frankfurt am Main 90 (DE); ANGENENDT, Heinrich, D-6000 Frankfurt am Main (DE)
(86) Internationale Anmeldenummer: EP9100359
(87) Internationale Veröffentlichungsnummer: WO9113866

(56) Entgegenhaltungen:
- EP-A- 0 141 776
- DE-A- 2 040 620
- US-A- 4 450 114

## Beschreibung

Die Erfindung betrifft ein Verfahren zur Herstellung von 3′-Aminopropyl-2-sulfatoethylsulfon in guten Ausbeuten, indem man in einem Eintopfverfahren Allylamin mit Mercaptoethanol in wäßriger Schwefelsäure bei Temperaturen von etwa 50°C bis zum Siedepunkt des Reaktionsgemisches in Gegenwart von Radikalstartern umsetzt, die angefallene Reaktionsmischung mit Wasserstoffperoxid in Anwesenheit katalytischer Mengen Übergangsmetall-Verbindungen oxidiert, dann mit Schwefelsäure versetzt und zur Trockene einengt.

3′-Aminopropyl-2-sulfatoethylsulfon ist ein wichtiges Vorprodukt zur Herstellung von Reaktivfarbstoffen (EP 0 141 776).

Die Herstellung von 3′-Aminopropyl-2-sulfatoethylsulfon wurde in der Literatur noch nicht beschrieben. Literaturbekannt ist jedoch die Herstellung von 2′-Aminoethyl-2-sulfatoethylsulfon. Hierbei wird nach der US-PS 2 824 887 2-Chlorethylamin-Hydrochlorid mit dem Natriumsalz von Mercaptoethanol umgesetzt und das so erhaltene 2′-Aminoethyl-2-hydroxyethylsulfid-Hydrochlorid mit Peressigsäure zum 2′-Aminoethyl-2-hydroxyethylsulfon-Hydrochlorid oxidiert.

In der EP-PS 0 141 776 (Seite 13) sind Methoden zur Sulfatierung von 2′-Aminoethyl-2-hydroxyethylsulfon-hydrochlorid bzw. ähnlichen Alkyl-hydroxyethylsulfonen mit Schwefelsäure bzw. Chlorsulfonsäure bei Temperaturen von 10 bis 80°C beschrieben. Wollte man 3′-Aminopropyl-2-sulfatoethylsulfon in analoger Weise herstellen, so wäre der Anfall von zwei Äquivalenten Natriumchlorid bei der Herstellung des 3′-Aminopropyl-2-hydroxyethylsulfids (Vorstufe) in Kauf zu nehmen. Ein verbessertes, ohne Salzanfall arbeitendes Verfahren zur Herstellung dieses Sulfides ist in der DE 2 040 620 beschrieben, indem Allylamin mit Mercaptoethanol unter Zusatz von Azoisobutyronitril (AIBN) bei 50 bis 150°C umgesetzt wird. In der zitierten Literaturstelle werden zu diesem Verfahren keine Ausbeuten angegeben. Eine Nacharbeitung des Verfahrens (Beispiel 4) ergab lediglich Ausbeuten von 56.6 % der Theorie. Somit ist festzuhalten, daß diese bekannten Verfahren zur Herstellung des 2′-Aminoethyl-2-sulfatoethylsulfons bzw. des 3′-Aminopropyl-2-hydroxyethylsulfids (Vorstufe) den Ansprüchen an ein technisches Verfahren nicht gerecht werden.

Es bestand daher ein Bedürfnis für ein wirtschaftliches und technisch leicht durchführbares Verfahren zur Herstellung von 3′-Aminopropyl-2-sulfatoethylsulfon.

Es wurde nun gefunden, daß man 3′-Aminopropyl-2-sulfatoethylsulfon in sehr guten Ausbeuten (> 95 % der Theorie) herstellen kann, indem man in einem Eintopfverfahren Allylamin mit Mercaptoethanol in verdünnter Schwefelsäure bei Temperaturen von etwa 50°C bis zum Siedepunkt, vorzugsweise von etwa 70°C bis etwa 100°C, in Gegenwart eines Radikalstarters umsetzt, die hierbei anfallende Reaktionsmischung mit Wasserstoffperoxid in Anwesenheit katalytischer Mengen einer Verbindung eines Übergangsmetalls des Periodischen Systems der Elemente, vorzugsweise einer Verbindung des Wolframs oder Vanadins, als Oxidationskatalysator bei Temperaturen von etwa 70 bis etwa 100°C, vorzugsweise von etwa 80 bis etwa 90°C oxidiert und nach Zusatz von soviel Schwefelsäure, daß die Gesamtschwefelsäuremenge im Gemisch mindestens 1 mol, bezogen auf das eingesetzte Allylamin, beträgt, zur Trockene einengt.

Das erfindungsgemäße Verfahren kann im einzelnen wie folgt durchgeführt werden: Man kann entweder so vorgehen, daß man beide Reaktionspartner in wäßriger Schwefelsäure mischt und in Gegenwart von Luft und/oder reinem Sauerstoff auf Reaktionstemperatur bringt, oder daß man Allylamin in etwa 0,5 bis etwa 0,7 mol wäßriger Schwefelsäure pro mol Allylamin vorlegt und Mercaptoethanol bei Temperaturen von etwa 50°C bis zum Siedepunkt des Reaktionsgemisches, bevorzugt etwa 70 bis etwa 100°C, zudosiert. Es ist auch möglich die Reaktion mit einem Radikalbildner aus der Reihe der Azoverbindungen, wie beispielsweise Azoisobutyronitril, oder der Peroxidverbindungen, wei beispielsweise Benzoylperoxid, zu starten. Das so erhaltene Reaktionsgemisch wird dann bei Temperaturen von etwa 70°C bis zum Siedepunkt der Reaktionsmischung mit einer katalytischen Menge einer beispielsweise Wolfram- oder Vanadin-Verbindung versetzt. Anschließend werden etwa 1,9 bis etwa 2,2 mol Wasserstoffperoxidlösung pro mol Allylamin bei diesen Temperaturen zudosiert. Nachdem etwa 1 h bei etwa 70 bis etwa 100°C nachgerührt worden ist, versetzt man mit etwa 0,3 bis etwa 0,7 mol Schwefelsäure, bezogen auf 1 mol eingesetztes Allylamin, so daß die Gesamt-Schwefelsäuremenge mindestens 1 mol, bezogen auf 1 mol eingesetztes Allylamin, beträgt und engt schließlich zur Trockene bei zuletzt etwa 150°C im Vakuum ein. Als Rückstand verbleibt 3′-Aminopropyl-2-sulfatoethylsulfon in hohen Ausbeuten von > 95 %.

Es ist zweckmäßig, 1 mol Allylamin mit etwa 0,9 bis etwa 1,5 mol, vorzugsweise mit etwa 0,95 bis etwa 1,05 mol Mercaptoethanol, in einer verbünnten Schwefelsäure, die zweckmäßigerweise etwa 10 bis etwa 50 gewichtsprozentig ist, reagieren zu lassen. Man kann auch eine weniger oder höher konzentrierte Schwefelsäure als Reaktionsmedium anwenden, jedoch ist bei Anwendung einer Schwefelsäure eine Konzentration von < 10 Gewichtsprozent eine schlechtere Raum-Zeit-Ausbeute in Kauf zu nehmen. Bei Anwendung einer höher konzentrierten Schwefelsäure können die Ausgangsverbindungen nicht mehr vollständig in Lösung gehen.

Was die eingesetzte Menge an Radikalstarter anbelangt, so wird in der Regel zunächst etwa 0,1 g pro mol eingesetztem Allylamin verwendet. Bei fortschreitender Reaktion wird dann etwa alle 4 Stunden jeweils etwa 0,1 g weiterer Radikalstarter pro mol eingesetztem Allylamin zugegeben. Größere Mengen an Radikalstarter können zwar zugesetzt werden, bringen aber praktisch keine weiteren Vorteile.

Die nach erfolgter Oxidation nochmals zugesetzte Schwefelsäure übersteigt zweckmäßigerweise 1,2 mol, bezogen auf eingesetztes Allylamin, nicht. Zwar kann auch eine größere Menge Schwefelsäure zugesetzt werden, doch ist damit keinerlei Vorteil verbunden.

Das erfindungsgemäße Verfahren wird zweckmäßigerweise bei Normaldruck durchgeführt; eine Verfahrensdurchführung bei erhöhtem Druck ist jedoch ebenfalls möglich.

Vorteilhaft an dem erfindungsgemäßen Verfahren ist, daß außer dem abdestillierten Wasser keine Abfallprodukte entstehen. Des weiteren ist es erstmals möglich, die Reaktion als Eintopfverfahren durchzuführen, wodurch das Verfahren aus ökonomischer und ökologischer Sicht sich sehr günstig gestaltet. Hierbei ist zwar die Oxidation mit Wasserstoffperoxid und die Veresterung mit Schwefelsäure prinzipiell bekannt, neu ist aber die radikalische Addition von Mercaptoethanol an Allylamin und die Kombination aller drei Verfahrensschritte zu einer Eintopfreaktion. Als überraschend ist zu werten, daß Allylamin mit Mercaptoethanol in wäßriger Schwefelsäure in Gegenwart von Luft und/oder Sauerstoff in hohen Ausbeuten reagiert, zumal aus der DE-PS 1 593 999 hervorgeht, daß bei Verwendung von oxidativen Radikalstartern bei der Umsetzung von Allylamin mit Mercaptopropionsäure unerwünschte Nebenprodukte entstehen.

Durch die nachstehenden Beispiele wird die Erfindung näher erläutert, ohne darauf beschränkt zu werden.

### Beispiel 1 (3′-Aminopropyl-2-sulfatoethylsulfon)

In einem 1-1-Vierhalskolben mit Rührer, Tropftrichter, Thermometer und Rückflußkühler werden 100,0 g Eis und 61,3 g (0,6 mol) Schwefelsäure 96 %ig vorgelegt. Dann läßt man 57,1 g (1,0 mol) Allylamin zulaufen. Anschließend wird das Gemisch mit 0,1 g Azoisobutyronitril (AIBN) versetzt und 78,1 g (1,0 mol) Mercaptoethanol zudosiert. Darauf wird bei 80 bis 85°C insgesamt 35 h nachgerührt, wobei im Abstand von jeweils 4 h jeweils weitere 0,1 g AIBN zudosiert werden. Nach beendeter Reaktion wird bei 80°C mit 1,0 g Natriumwolframat-dihydrat versetzt und innerhalb von 1 h werden 113,3 g (1,0 mol) Wasserstoffperoxid 30%ig zudosiert. Um die Temperatur von 80°C zu halten, muß mit Eiswasser gekühlt werden. Dann werden nochmals 113,3 g (1,0 mol) Wasserstoffperoxid 30%ig bei 80°C zudosiert. Nach beendeter Dosierung wird 1 h bei 80°C nachgerührt. Zur Veresterung des Oxethylsulfons werden 46,0 g (0,45 mol) Schwefelsäure 96%ig zugegeben. Bei einer Temperatur von 80°C/200 mbar wird die Reaktionsmischung in einen Laborkneter getropft und dann die Temperatur langsam auf 150°C/l mbar gesteigert. Schließlich wird zur Trockene eindestilliert. Es fallen 254,7 g 3′-Aminopropyl-2-sulfatoethylsulfon mit einem Reingehalt von 93.6 % an, was einer Ausbeute von 96,4 % d.Th. entspricht.

Schmelzpunkt: 235 - 240°C (Zersetzung)
¹H-NMR ([D₆]DMSO): δ = 2,0 (q, J=7Hz;CH₂CH₂CH₂CH₂;2H),
2,9 (m;CH₂NH₃⁺;2H), 3,2 (m;SO₂CH₂CH₂;2H),
3,4 (t, J=7Hz;CH₂CH₂SO₂), 4,1 (t, J=7Hz;CH₂OSO₃⁻;2H),
7,7 (breit;NH₃⁺;3H).
IR (KBr): 3160, 2990, 2935, 1320, 1290, 1205, 1060 cm⁻¹.

### Beispiel 2 (3′-Aminopropyl-2-sulfatoethylsulfon)

In einem 1-1-Vierhalskolben mit Rührer, Tropftrichter, Thermometer und Rückflußkühler werden 200,0 g Eis und 51,1 g (0,5 mol) Schwefelsäure 96 %ig vorgelegt. Man läßt nun 57,1 g (1,0 mol) Allylamin zulaufen. Anschließend werden 78,1 g (1,0 mol) Mercaptoethanol zudosiert. Dann wird bei 100°C insgesamt 40 h nachgerührt und gleichzeitig Luft durch das Reaktionsgemisch geleitet. Nach beendeter Reaktion wird bei 80°C mit 0,5 g Natriumwolframat-dihydrat versetzt und innerhalb von 1 h werden 113,3 g (1,0 mol) Wasserstoffperoxid 30%ig zudosiert. Um die Temperatur von 80°C zu halten, muß mit Eiswasser gekühlt werden. Dann werden nochmals 113,3 g (1,0 mol) Wasserstoffperoxid 30%ig bei 80°C zudosiert. Nach beendeter Dosierung wird 1 h bei 80°C nachgerührt. Zur Veresterung des Oxethylsulfons werden 56,3 g (0,55 mol) Schwefelsäure 96%ig zugegeben. Bei einer Temperatur von 80°C/200 mbar wird die Reaktionsmischung in einen Laborkneter getropft und dann die Temperatur langsam auf 150°C/l mbar gesteigert. Schließlich wird zur Trockene eindestilliert. Es fallen 249,5 g 3′-Aminopropyl-2-sulfatoethylsulfon mit einem Reingehalt von 96,5 % an, was einer Ausbeute von 97,3 % d.Th. entspricht.

Schmelzpunkt: 235-240°C (Zersetzung)
Die spektroskopischen Daten sind mit denen, die am Ende des Beispiels 1 angegeben sind, identisch.

### Beispiel 3 (3′-Aminopropyl-2-sulfatoethylsulfon)

In einem 1-1-Vierhalskolben mit Rührer, Tropftrichter, Thermometer und Rückflußkühler werden 100,0 g Eis und 56,3 g (0,55 mol) Schwefelsäure 96 %ig vorgelegt. Dann gibt man 57,1 g (1,0 mol) Allylamin und 78,1 g (1,0 mol) Mercaptoethanol zu. Die erhaltene Reaktionsmischung wird mit 0,1 g Dibenzoylperoxid versetzt und bei 80 bis 85°C insgesamt 45 h nachgerührt, wobei nach jeweils 5 h jeweils 0,1 g Benzoylperoxid zugegeben wird. Nach beendeter Reaktion wird bei 80°C mit 1,0 g Natriumwolframat-dihydrat versetzt. Dann werden innerhalb von 2 h 226,6 g (1,0 mol) Wasserstoffperoxid 30%ig zudosiert. Nach beendeter Dosierung wird 1 h bei 80°C nachgerührt. Zur Veresterung des Oxethylsulfons werden 56,3 g (0,55 mol) Schwefelsäure 96 %ig zugegeben. Bei einer Temperatur von 80°C/200 mbar wird das Reaktionsgemisch in einen Laborkneter getropft und dann die Temperatur langsam auf 150°C/l mbar gesteigert. Schließlich wird zur Trockene durch Destillation eingeengt. Es fallen 256,5 g 3′-Aminopropyl-2-sulfatoethylsulfon mit einem Reingehalt von 91,8 % an, was einer Ausbeute von 94,6 % d.Th. entspricht.

Schmelzpunkt: 235-240°C (Zersetzung).
Die spektroskopischen Daten sind mit denen, die am Ende des Beispiels 1 angegeben sind, identisch.

Ersetzt man das Natriumwolframat-dihydrat durch die gleiche Menge Natriummetavanadat (NaVO₃) und arbeitet im übrigen wie vorstehend angegeben, so kommt man zum gleichen Ergebnis.

### Beispiel 4 (3′-Aminopropyl-2-hydroxyethylsulfid) -Vergleichsbeispiel nach Angaben des Beispiels 3, Seite 18, der DE 2 040 620 -

Zu 78,1 g (1,0 mol) Mercaptoethanol, das mit 0,5 g AIBN versetzt ist, werden bei 80 bis 90°C über 2 h 57,0 g (1,0 mol) Allylamin getropft. Dann wird 10 h bei 80 bis 90°C nachgerührt. Es fallen 126.8 g 3′-Aminopropyl-2-hydroxyethylsulfid mit einer Reinheit von 60,4 % an, was einer Ausbeute von 56,6 % d. Th. entspricht.

## Patentansprüche

1. Verfahren zur Herstellung von 3′-Aminopropyl-2-sulfatoethylsulfon, dadurch gekennzeichnet, daß man in einem Eintopfverfahren Allylamin mit Mercaptoethanol in verdünnter Schwefelsäure bei Temperaturen von etwa 50°C bis zum Siedepunkt in Gegenwart eines Radikalstarters umsetzt, die hierbei anfallende Reaktionsmischung mit Wasserstoffperoxid in Anwesenheit katalytischer Mengen einer Verbindung eines Übergangsmetalls des Periodischen Systems der Elemente als Oxidationskatalysator bei Temperaturen von etwa 70 bis etwa 100°C oxidiert und nach Zusatz von soviel Schwefelsäure, daß die Gesamtschwefelsäuremenge im Gemisch mindestens 1 mol, bezogen auf das eingesetzte Allylamin, beträgt, zur Trockene einengt.

2. Verfahren nach Anspruch 1, dadurch gekennzeichnet, daß man das Allylamin mit dem Mercaptoethanol in der wäßrigen Schwefelsäure bei Temperaturen von etwa 70 bis etwa 100°C umsetzt.

3. Verfahren nach mindestens einem der Ansprüche 1 und 2, dadurch gekennzeichnet, daß man in Gegenwart von reinem Sauerstoff als Radikalstarter umsetzt.

4. Verfahren nach mindestens einem der Ansprüche 1 und 2, dadurch gekennzeichnet, daß man in Gegenwart einer Mischung aus Sauerstoff und einem Inertgas als Radikalstarter umsetzt.

5. Verfahren nach mindestens einem der Ansprüche 1, 2 und 4, dadurch gekennzeichnet, daß man in Gegenwart von Luft als Radikalstarter umsetzt.

6. Verfahren nach mindestens einem der Ansprüche 1 und 2, dadurch gekennzeichnet, daß man in Gegenwart einer Azoverbindung als Radikalstarter umsetzt.

7. Verfahren nach mindestens einem der Ansprüche 1, 2 und 6, dadurch gekennzeichnet, daß man in Gegenwart von Azoisobutyronitril als Radikalstarter umsetzt.

8. Verfahren nach mindestens einem der Ansprüche 1 und 2, dadurch gekennzeichnet, daß man in Gegenwart einer Peroxidverbindung als Radikalstarter umsetzt.

9. Verfahren nach mindestens einem der Ansprüche 1, 2 und 8, dadurch gekennzeichnet, daß man in Gegenwart von Benzoylperoxid als Radikalstarter umsetzt.

10. Verfahren nach mindestens einem der Ansprüche 1 bis 9, dadurch gekennzeichnet, daß man in Gegenwart einer Verbindung des Wolframs oder Vanadins als Oxidationskatalysator oxidiert.

11. Verfahren nach mindestens einem der Ansprüche 1 bis 10, dadurch gekennzeichnet, daß man in Gegenwart von Na₂WO₄·2H₂O als Oxidationskatalysator oxidiert.

12. Verfahen nach mindestens einem der Ansprüche 1 bis 10, dadurch gekennzeichnet, daß man in Gegenwart von NaVO₃ als Oxidationskatalysator oxidiert.

13. Verfahren nach mindestens einem der Ansprüche 1 bis 12, dadurch gekennzeichnet, daß man bei Normaldruck oder Überdruck umsetzt.

## Claims

1. A process for the preparation of 3-aminopropyl 2-sulfatoethyl sulfone, which comprises reacting in a one-pot process allylamine with mercaptoethanol in dilute sulfuric acid at temperatures of about 50°C to the boiling point in the presence of a free-radical initiator, oxidizing the resulting reaction mixture with hydrogen peroxide in the presence of catalytic amounts of a compound of a transition metal of the periodic table of the elements as the oxidation catalyst at temperatures of about 70 to about 100°C, and, after sulfuric acid has been added in such an amount that the total amount of sulfuric acid in the mixture is at least 1 mol, relative to the allylamine used, evaporating the mixture to dryness.

2. The process as claimed in claim 1, wherein the allylamine is reacted with the mercaptoethanol in the aqueous sulfuric acid at temperatures of about 70 to about 100°C.

3. The process as claimed in at least one of claims 1 and 2, wherein the reaction is carried out in the presence of pure oxygen as the free-radical initiator.

4. The process as claimed in at least one of claims 1 and 2, wherein the reaction is carried out in the presence of a mixture of oxygen and an inert gas as the free-radical initiator.

5. The process as claimed in at least one of claims 1, 2 and 4, wherein the reaction is carried out in the presence of air as the free-radical initiator.

6. The process as claimed in at least one of claims 1 and 2, wherein the reaction is carried out in the presence of an azo compound as the free-radical initiator.

7. The process as claimed in at least one of claims 1, 2 and 6, wherein the reaction is carried out in the presence of azobisisobutyronitrile as the free-radical initiator.

8. The process as claimed in at least one of claims 1 and 2, wherein the reaction is carried out in the presence of a peroxide compound as the free-radical initiator.

9. The process as claimed in at least one of claims 1, 2 and 8, wherein the reaction is carried out in the presence of benzoyl peroxide as the free-radical initiator.

10. The process as claimed in at least one of claims 1 to 9, wherein the oxidation is carried out in the presence of a compound of tungsten or vanadium as the oxidation catalyst.

11. The process as claimed in at least one of claims 1 to 10, wherein the oxidation is carried out in the presence of Na₂WO₄ × 2H₂O as the oxidation catalyst.

12. The process as claimed in at least one of claims 1 to 10, wherein the oxidation is carried out in the presence of NaVO₃ as the oxidation catalyst.

13. The process as claimed in at least one of claims 1 to 12, wherein the reaction is carried out at atmospheric pressure or superatmospheric pressure.

## Revendications

1. Procédé pour préparer la 3′-aminopropyl-2-sulfatoéthylsulfone, caractérisé en ce que, dans un procédé en un pot, on fait réagir de l'allylamine avec du mercaptoéthanol dans de l'acide sulfurique dilué à des températures comprises entre environ 50°C et le point d'ébullition en présence d'un amorceur radicalaire, on oxyde le mélange réactionnel ainsi obtenu avec du peroxyde d'hydrogène en présence de quantités catalytiques d'un composé d'un métal de transition du Tableau Périodique des éléments, servant de catalyseur d'oxydation, à des températures comprises entre environ 70 et environ 100°C, et que, après addition d'une quantité d'acide sulfurique suffisante pour que la quantité totale d'acide sulfurique dans le mélange soit d'au moins 1 mol par rapport à la quantité utilisée d'allylamine, on concentre jusqu'à siccité.

2. Procédé selon la revendication 1, caractérisé en ce qu'on fait réagir l'allylamine avec le mercaptoéthanol dans l'acide sulfurique en solution aqueuse à des températures d'environ 70 à environ 100°C.

3. Procédé selon au moins l'une des revendications 1 et 2, caractérisé en ce qu'on met en oeuvre la réaction en présence d'oxygène pur servant d'amorceur radicalaire.

4. Procédé selon au moins l'une des revendications 1 et 2, caractérisé en ce qu'on met en oeuvre la réaction en présence d'un mélange d'oxygène et d'un gaz inerte servant d'amorceur radicalaire.

5. Procédé selon au moins l'une des revendications 1, 2 et 4, caractérisé en ce qu'on met en oeuvre la réaction en présence d'air servant d'amorceur radicalaire.

6. Procédé selon au moins l'une des revendications 1 et 2, caractérisé en ce qu'on met en oeuvre la réaction en présence d'un composé azoïque servant d'amorceur radicalaire.

7. Procédé selon au moins l'une des revendications 1, 2 et 6, caractérisé en ce qu'on met en oeuvre la réaction en présence d'azoisobutyronitrile servant d'amorceur radicalaire.

8. Procédé selon au moins l'une des revendications 1 et 2, caractérisé en ce qu'on met en oeuvre la réaction en présence d'un peroxyde servant d'amorceur radicalaire.

9. Procédé selon au moins l'une des revendications 1, 2 et 8, caractérisé en ce qu'on met en oeuvre la réaction en présence de peroxyde de benzoyle servant d'amorceur radicalaire.

10. Procédé selon au moins l'une des revendications 1 à 9, caractérisé en ce qu'on met en oeuvre l'oxydation en présence d'un composé du tungstène ou du vanadium servant de catalyseur d'oxydation.

11. Procédé selon au moins l'une des revendications 1 à 10, caractérisé en ce qu'on met en oeuvre l'oxydation en présence de Na₂WO₄·2H₂O servant de catalyseur d'oxydation.

12. Procédé selon au moins l'une des revendications 1 à 10, caractérisé en ce qu'on met en oeuvre l'oxydation en présence de NaVO₃ servant de catalyseur d'oxydation.

13. Procédé selon au moins l'une des revendications 1 à 12, caractérisé en ce qu'on met en oeuvre la réaction sous la pression normale ou en surpression.
